# FASCICULE DE BREVET EUROPEEN

(11) **EP 0 659 398 B1**
(45) Date de publication et mention de la délivrance du brevet: **09.04.1997**
(21) Numéro de dépôt: 94402570.9
(22) Date de dépôt: 14.11.1994
(51) Int. Cl.: A61K 7/13, A45D 7/00, A45D 19/00

(54) **Procédé de coloration directe des fibres kératiniques humaines à l'aide de colorants sulfoniques et de vapeur d'eau**
Verfahren zum direkten Haarfärben unter Anwendung von Sulfogruppenhaltige Farbstoffen und Wasserdampf
Process of direct hair dyeing using sulphonic groups containing dyes and steam

(30) Priorité: 22.12.1993 FR 9315483
(43) Date de publication de la demande: 28.06.1995
(73) Titulaire: L'OREAL, 75008 Paris (FR)
(72) Inventeur: Samain, Henri, F-91570 Bievres (FR); Sturla, Jean-Michel, F-92210 Saint-Cloud (FR)
(74) Mandataire: Andral, Christophe André Louis

(56) Documents cités:
- EP-A- 0 103 547
- DE-U- 9 318 614
- FR-A- 1 157 665
- FR-A- 2 273 492
- US-A- 3 630 654

## Description

La présente invention est relative à un procédé de coloration (ou teinture) directe des fibres kératiniques en particulier des cheveux, mettant en oeuvre de la vapeur d'eau et une composition comprenant des colorants directs à fonction sulfonique.

Pour réaliser des coloration semi-permanentes, on utilise des colorants dits directs c'est à dire des colorants qui ne mettent pas en oeuvre de mécanisme d'oxydation et qui sont capables de colorer par eux-mêmes les fibres kératiniques.

De tels colorants, grâce à la variété des substances qu'il est possible de mettre en oeuvre, permettent de couvrir une large gamme de nuances allant du jaune au bleu en passant par le rouge.

Parmi ces colorants directs, on connaît les colorants portant des groupements sulfoniques. Ces colorants sont particulièrement intéressants puisqu'ils permettent d'obtenir des résultats tinctoriaux présentant une forte ténacité. Les cheveux ainsi traités gardent longtemps l'effet de la coloration.

Cependant, ils présentent un inconvénient majeur limitant leur utilisation. En effet, ces colorants à fonction sulfonique tachent très facilement la peau et en particulier le cuir chevelu. Ainsi, si de la composition colorante a malencontreusement été appliquée ou a coulé sur le cuir chevelu, celui-ci se trouve, en fin d'opération de coloration de la chevelure (laquelle, habituellement, nécessite plusieurs dizaines de minutes de temps de pause), fortement et durablement taché.

A ce jour, seule l'habileté du coiffeur permet d'éliminer ou de limiter l'effet indésirable ci-dessus.

La présente invention a pour but de résoudre le problème ci-dessus en proposant un procédé fiable, simple, efficace et de mise en oeuvre aisée, de coloration directe des fibres kératiniques qui permette de s'affranchir des inconvénients inhérents à l'emploi des colorants sulfoniques, tout en en conservant les avantages.

La demanderesse a maintenant découvert de façon surprenante que l'utilisation d'un gaz chauffé à une température supérieure à 75°C comprenant de la vapeur d'eau sur des cheveux traités avec des colorants directs à fonction sulfonique permettait d'obtenir d'excellents résultats tinctoriaux en des temps extrêmement limités et donc sans risque de tachage de la peau ou du cuir chevelu.

Les cheveux présentent en outre d'excellentes qualités cosmétiques finales.

Ce procédé permet également d'utiliser les colorants sulfoniques dans des gammes de pH plus étendues, et en particulier à pH plus basique, que dans les procédés de l'art antérieur.

La présente invention concerne donc un procédé de teinture directe des fibres kératiniques, caractérisé par le fait qu'il consiste à mettre en contact des fibres kératiniques sur lesquelles on a appliqué une composition contenant au moins un colorant direct portant au moins une fonction sulfonique, avec un gaz contenant de la vapeur d'eau, la température dudit gaz étant supérieure à 75°C, le temps de contact entre ledit gaz et lesdites fibres à colorer étant inférieur à deux minutes.

Les excellents résultats procurés par l'invention sont d'autant plus surprenants qu'il est connu que les colorants sulfoniques sont sensibles à la chaleur et que l'effet de tachage évoqué précédemment se développe d'autant plus rapidement que la température est élevée ; l'homme de l'art n'était donc pas enclin à mettre en oeuvre dans un procédé à la vapeur des colorants sulfoniques conformes à l'invention.

Les colorants à fonction sulfonique peuvent être choisis parmi les composés suivants :

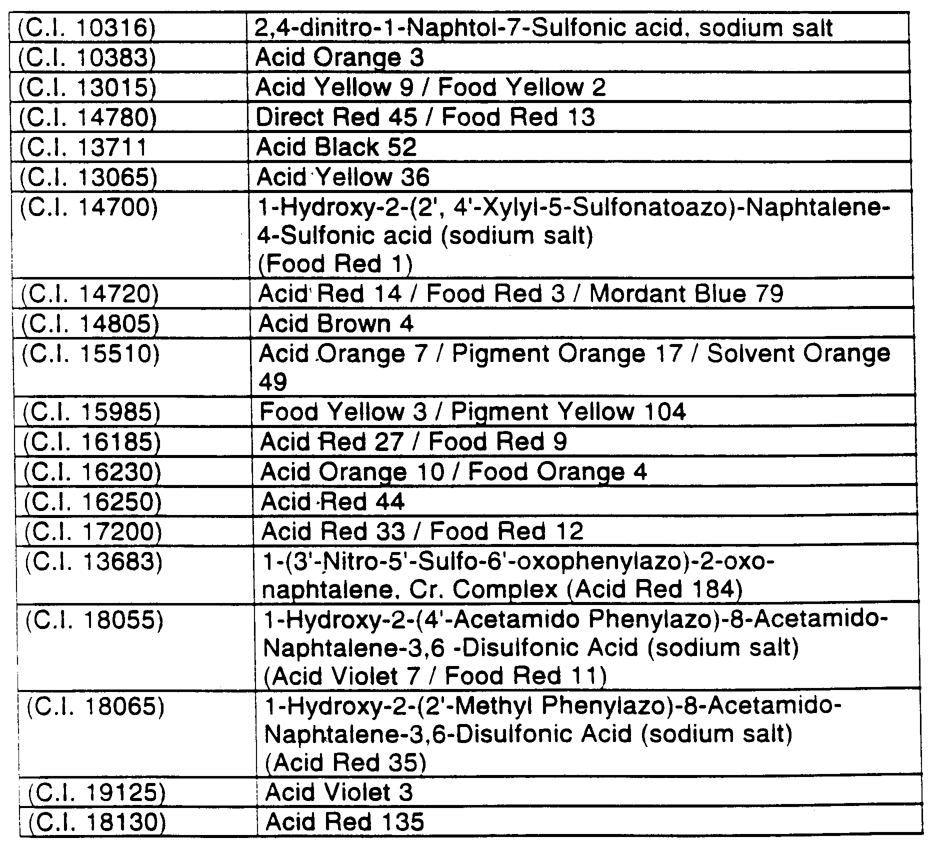

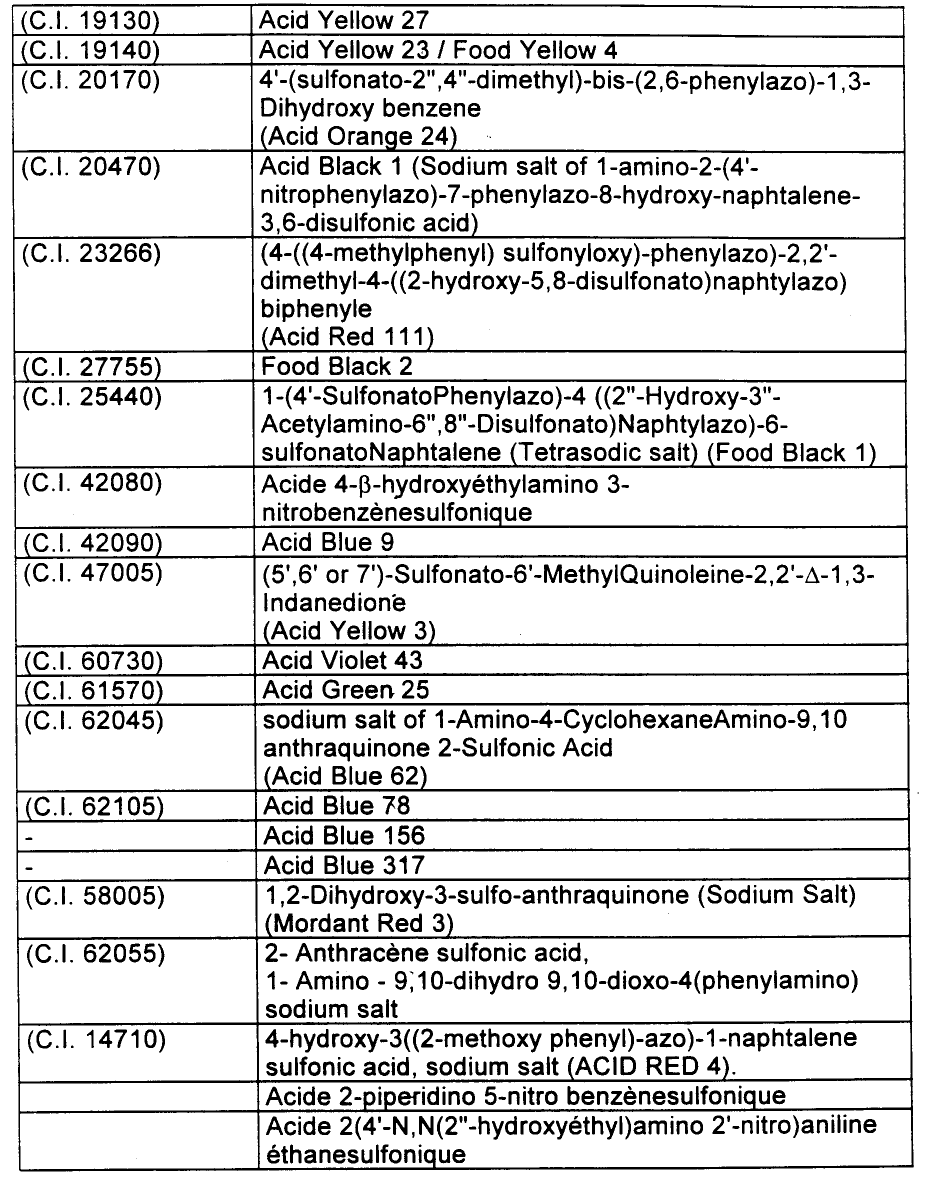

La plupart de ces colorants sont décrits en particulier dans le Color Index (publié par The Society of Dyers and Colorists, P.O. Box 244, Perkin House, 82 Grattan Road, Bradford, Yorkshire, BD1 2JB, ENGLAND).

Les colorants sulfoniques plus particulièrement préférés sont les colorants désignés sous le code C.I. 60730, C.I. 15510, C.I. 47005, C.I. 15985, C.I. 17200,C.I. 20470, C.I. 42090, C.I. 61570 dans le Color Index.

Le ou les colorants directs à fonction sulfonique sont généralement présents dans des concentrations allant de 0,01 à 10% en poids par rapport au poids total de la composition.

En plus de la vapeur d'eau, le gaz vecteur peut contenir de la vapeur de solvant, des gaz tels que l'oxygène, l'azote, des mélange de gaz tels que l'air ou d'autres composés vaporisables.

Les solvants utilisables pour la production de vapeur sont des solvants organiques cosmétiquement acceptables et plus particulièrement des alcools tels que l'éthanol, l'isopropanol, l'alcool benzylique, l'alcool phényléthylique ou des glycols ou éthers de glycol tels que par exemple les éthers monométhylique, monoéthylique et monobutylique de l'éthylène glycol, le propylène glycol, le butylène glycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit d'un mélange de vapeur d'eau et d'air.

La température du gaz est de préférence supérieure ou égale à 85°C et plus particulièrement comprise entre 85 et 150°C.

Le gaz est mis en contact avec la fibre à colorer pendant une durée allant de 0,01 seconde à 2 minutes, de préférence de 0,01 seconde à 30 secondes et encore plus préférentiellement de 0,5 à 10 secondes. L'application du gaz peut être répétée plusieurs fois sur la même fibre, chaque opération se faisant selon la durée indiquée ci-dessus.

Dans un premier mode de mise en oeuvre du procédé selon l'invention qui est ici préféré, on applique sur les cheveux une composition de coloration capillaire contenant les colorants directs sulfoniques, puis on les soumet à l'action de la vapeur d'eau.

Selon d'autres modes de mise en oeuvre du procédé, il est également possible d'appliquer simultanément la composition colorante et le gaz comprenant de la vapeur d'eau.

Il est également possible de faire parvenir sur les cheveux tout ou partie de la composition colorante à l'aide du flux de gaz lorsque certains ou tous les constituants de la formule sont entraînables ou vaporisables.

Dans un mode particulier de réalisation de l'invention, l'application de vapeur d'eau est suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans la demande de brevet français FR-A-2273492, ou tout autre appareil équivalent, qui convient particulièrement bien (traitement ponctuel, régulier et homogène des fibres, sans risque de surchauffe).

Compte tenu des temps de pause particulièrement courts pour développer les teintures sur cheveux selon l'invention, si du colorant a été mis en contact avec la peau et/ou le cuir chevelu, accidentellement ou en raison d'une mauvaise manipulation, celui-ci n'a pas le temps de s'absorber, ce qui élimine tout risque de tachage.

La composition tinctoriale utilisée dans le procédé selon l'invention peut se présenter sous des formes habituellement utilisées pour la teinture des cheveux telle que de liquide plus ou moins épaissi ou gélifié, de crème, de mousse en aérosol ou sous toute autre forme appropriée pour réaliser une teinture des cheveux.

Les compositions utilisées conformément à l'invention sont généralement des compositions aqueuses pouvant contenir des ingrédient habituellement utilisés dans les compositions cosmétiques destinées à la coloration des cheveux, tels que des solvants, des agents tensioactifs, des épaississants, des agents traitants, des agents alcalinisants ou acidifiants, des agents conservateurs, des parfums ou tout autre additif utilisé dans ce type de composition.

La composition tinctoriale contenant au moins un colorant à fonction sulfonique présente un pH généralement compris entre 2 et 11.

L'exemple qui suit illustre l'invention sans pour autant présenter un caractère limitatif.

### EXEMPLE:

On utilise une composition tinctoriale présentant les caractéristiques suivantes:

| | |
|---|---|
| - Acid black 1 | 0,4 g |
| - Alcool benzylique | 3 g |
| - 1,3-butylèneglycol | 10 g |
| - Gomme de xanthane | 1,5 g |
| - Acide citrique qs | pH 3,5 |
| - Eau qsp | 100 g |

Le mode opératoire est le suivant : on applique la composition ci-dessus sur des cheveux et on laisse volontairement couler de la composition sur la peau.

Sur une première partie de la chevelure, on envoie ensuite un jet de vapeur d'eau à 90°C pendant 5 secondes. On rince à l'eau les cheveux et la peau puis on sèche (peau n°1).
Sur l'autre partie de la chevelure, on laisse par contre poser pendant 30 minutes à température ordinaire. On rince à l'eau les cheveux et la peau puis on sèche (peau n°2).

Dans les deux cas, on mesure les coordonnées chromatiques L, a, b de la peau avant et après la teinture (colorimètre MINOLTA CHROMA METER CR 200), ce qui permet de comparer la différence de tachage entre les deux procédés.
ΔL, Δa et Δb représentent les différences, en valeurs absolues, entre les valeurs de L, a et b finales (après coloration) et les valeurs de L, a et b initiales (avant la coloration) de la peau.

| | ΔL | Δa | Δb |
|---|---|---|---|
| peau n°1 | 2,6 | 1,45 | 1,9 |
| peau n°2 | 14,2 | 8,5 | 10,2 |

On remarque qu'avec l'utilisation du procédé à la vapeur selon l'invention, la peau est nettement moins tachée. Les ΔL, Δa et Δb sont en effet beaucoup plus faibles que ceux obtenus avec le procédé classique.

## Revendications

1. Procédé de teinture directe des fibres kératiniques sans risque de tachage de la peau ou du cuir chevelu, caractérisé par le fait qu'il consiste à mettre en contact des fibres kératiniques sur lesquelles on a appliqué une composition contenant au moins un colorant direct à fonction sulfonique, avec un gaz contenant de la vapeur d'eau, la température dudit gaz étant supérieure à 75°C, le temps de contact entre ledit gaz et lesdites fibres à colorer étant inférieur à deux minutes.

2. Procédé selon la revendication 1, caractérisé par le fait que le gaz a une température supérieure ou égale à 85°C.

3. Procédé selon la revendication 2, caractérisé par le fait que le gaz a une température comprise entre 85 et 150°C.

4. Procédé selon l'une quelconque des revendications précédentes, caractérisé par le fait que le gaz est mis en contact avec la fibre à colorer pendant une durée allant de 0,01 seconde à 2 minutes.

5. Procédé selon la revendication 4, caractérisé par le fait que le gaz est mis en contact avec la fibre à colorer pendant une durée allant de 0,01 seconde à 30 secondes.

6. Procédé selon la revendication 5, caractérisé par le fait que le gaz est mis en contact avec la fibre à colorer pendant une durée allant de 0,5 seconde à 10 secondes.

7. Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que le gaz contient uniquement de la vapeur d'eau.

9. Procédé selon l'une quelconque des revendications 1 à 7, caractérisé par le fait que le gaz contient de la vapeur d'eau et au moins un autre composé sous forme de gaz ou de vapeur.

10. Procédé selon la revendication 9, caractérisé par le fait que le gaz contient de la vapeur d'eau et de l'air.

11. Procédé selon l'une quelconque des revendications précédentes, caractérisé par le fait que les colorants directs à fonction sulfonique sont choisis parmi les composés désignés sous le code C.I. 60730, C.I. 15510, C.I. 47005, C.I. 15985, C.I. 17200,C.I. 20470, C.I. 42090, C.I. 61570 dans le Color Index.

12. Procédé selon l'une quelconque des revendications précédentes, caractérisé par le fait que les colorants directs à fonction sulfonique sont présents dans des concentrations allant de 0,01 à 10% en poids par rapport au poids total de la composition.

13. Procédé selon l'une quelconque des revendications précédentes, caractérisé par le fait que la composition contenant au moins un colorant direct à fonction sulfonique présente un pH compris entre 2 et 11.

## Claims

1. Process for directly dyeing keratinous fibres without risk of staining the skin or the scalp, characterized in that it consists in bringing keratinous fibres, to which a composition containing at least one direct dye containing a sulphonic functional group has been applied, into contact with a gas containing water vapour, the temperature of the said gas being greater than 75°C and the contact time between the said gas and the said fibres to be dyed being less than two minutes.

2. Process according to Claim 1, characterized in that the gas has a temperature greater than or equal to 85°C.

3. Process according to Claim 2, characterized in that the gas has a temperature between 85 and 150°C.

4. Process according to any one of the preceding claims, characterized in that the gas is brought into contact with the fibre to be dyed for a period of time ranging from 0.01 second to 2 minutes.

5. Process according to Claim 4, characterized in that the gas is brought into contact with the fibre to be dyed for a period of time ranging from 0.01 second to 30 seconds.

6. Process according to Claim 5, characterized in that the gas is brought into contact with the fibre to be dyed for a period of time ranging from 0.5 second to 10 seconds.

7. Process according to any one of the preceding claims, characterized in that application of the gas is repeated a number of times on the same fibre.

8. Process according to any one of the preceding claims, characterized in that the gas contains solely water vapour.

9. Process according to any one of Claims 1 to 7, characterized in that the gas contains water vapour and at least one other compound in the form of gas or vapour.

10. Process according to Claim 9, characterized in that the gas contains water vapour and air.

11. Process according to any one of the preceding claims, characterized in that the direct dyes containing sulphonic functional groups are chosen from the compounds denoted under the code C.I. 60730, C.I. 15510, C.I. 47005, C.I. 15985, C.I. 17200, C.I. 20470, C.I. 42090 and C.I. 61570 in the Colour Index.

12. Process according to any one of the preceding claims, characterized in that the direct dyes containing sulphonic functional groups are present in concentrations ranging from 0.01 to 10 % by weight with respect to the total weight of the composition.

13. Process according to any one of the preceding claims, characterized in that the composition containing at least one direct dye containing a sulphonic functional group has a pH of between 2 and 11.

## Patentansprüche

1. Verfahren zum direkten Färben von Keratinfasern ohne Risiko der Fleckenbildung auf der Haut oder auf der Kopfhaut,
dadurch gekennzeichnet, daß
es darin besteht, Keratinfasern, auf die eine Zusammensetzung, die mindestens einen Direktfarbstoff mit Sulfonsäuregruppe enthält, aufgetragen wurde, mit einem Gas, das Wasserdampf enthält, in Kontakt zu bringen, wobei die Temperatur des Gases über 75 °C liegt und die Kontaktzeit zwischen dem Gas und den zu färbenden Fasern weniger als 2 min beträgt.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß das Gas eine Temperatur im Bereich von 85 bis 150 °C aufweist.

4. Verfahren nach einem der vorhergehenden Ansprüche, dadurch gekennzeichnet, daß das Gas mit der zu färbenden Faser während einer Zeitdauer von 0,01 s bis 2 min in Kontakt gebracht wird.

5. Verfahren nach Anspruch 4, dadurch gekennzeichnet, daß das Gas mit der zu färbenden Faser für eine Zeitdauer von 0,01 bis 30 s in Kontakt gebracht wird.

6. Verfahren nach Anspruch 5, dadurch gekennzeichnet, daß das Gas mit der zu färbenden Faser für eine Zeitdauer von 0,5 bis 10 s in Kontakt gebracht wird.

7. Verfahren nach einem der vorhergehenden Ansprüche, dadurch gekennzeichnet, daß die Anwendung des Gases mehrmals an der gleichen Faser wiederholt wird.

8. Verfahren nach einem der vorhergehenden Ansprüche, dadurch gekennzeichnet, daß das Gas nur Wasserdampf enthält.

9. Verfahren nach einem der Ansprüche 1 bis 7, dadurch gekennzeichnet, daß das Gas Wasserdampf und mindestens eine weitere Verbindung in Form von Gas oder Dampf enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, daß die Direktfarbstoffe mit Sulfonsäuregruppen unter den Verbindungen ausgewählt sind, die mit den Color-Index-Nummern C.I. 60730, C.I. 15510, C.I. 47005, C.I. 15985, C.I. 17200, C.I. 20470, C.I. 42090 und C.I. 61570 bezeichnet sind.

12. Verfahren nach einem der vorhergehenden Ansprüche, dadurch gekennzeichnet, daß die Direktfarbstoffe mit Sulfonsäuregruppen in Konzentrationen im Bereich von 0,01 bis 10 Gew.-%, bezogen auf das Gesamtgewicht der Zusammensetzung, vorliegen.

13. Verfahren nach einem der vorhergehenden Ansprüche, dadurch gekennzeichnet, daß die Zusammensetzung, die mindestens einen Direktfarbstoff mit Sulfonsäuregruppen enthält, einen pH-Wert von 2 bis 11 aufweist.
